# EUROPEAN PATENT SPECIFICATION

(11) **EP 3 442 484 B1**
(45) Date of publication and mention of the grant of the patent: **23.08.2023**
(21) Application number: 17782896.9
(22) Date of filing: 10.04.2017
(51) Int. Cl.: A61F 9/007, A61F 2/856, A61F 2/94, A61M 27/00

(54) **LACRIMAL STENT WITH OPENING**
LAKRIMALSTENT MIT ÖFFNUNG
ENDOPROTHÈSE LACRYMALE DOTÉE D'UNE OUVERTURE

(30) Priority: 11.04.2016 US 201662320789 P
(43) Date of publication of application: 20.02.2019
(73) Proprietor: The Regents of The University of Michigan, Ann Arbor, MI 48109-2590 (US)
(72) Inventor: KAHANA, Alon, Ann Arbor, Michigan 48108 (US); PLOTT, Jeffrey Stephen, Clay Township, Michigan 48001 (US)
(74) Representative: Witte, Weller & Partner Patentanwälte mbB
(86) International application number: PCT/US2017/026740
(87) International publication number: WO 2017/180487

(56) References cited:
- WO-A1-2011/075481
- WO-A1-2014/150292
- WO-A1-2015/002510
- AT-B- 316 011
- CN-Y- 201 164 535
- JP-A- 2006 181 054
- US-A- 5 336 177
- US-A1- 2008 082 037
- US-A1- 2014 364 790

## Description

### CROSS-REFERENCE TO RELATED APPLICATIONS

This application claims the benefit of U.S. Provisional Application No. 62/320,789, filed on April 11, 2016.

### FIELD

The present disclosure relates to a lacrimal stent with opening.

### BACKGROUND AND SUMMARY

This section provides background information related to the present disclosure which is not necessarily prior art. This section also provides a general summary of the disclosure, and is not a comprehensive disclosure of its full scope or all of its features.

Stents are implantable medical devices used to maintain patency of vessels in the body; that is, the recanalization of obstructed or structurally compromised vascular and nonvascular circulatory ducts in various organs and tissues of the human body.

Every year 16% to 36% cases of eyelid trauma cause injury to the lacrimal system, and epiphora accounting for nearly 3% of clinic visits. On average, the cost of each ocular stent is approximately $500.

Epiphora, the imperfect draining of tears through the lacrimal passage, is a common ophthalmic problem. Abnormalities of tear drainage may be subdivided further into functional and anatomical issues. Functional failure is related to poor lacrimal pump function, which may be due to a displaced punctum, eyelid laxity, weak orbicularis, or cranial nerve VII palsy. Anatomical obstruction may occur at any point along the lacrimal drainage pathway and may be congenital or acquired due to trauma.

Epiphora can affect patient quality of life (QoL) by causing blurred vision, discharge, sore skin, and/or a persistent crying appearance. In some extreme cases, the stagnating tears may result in bacterial growth, fungi, and viruses, resulting in secondary complications, such as inflammation and infection.

The difference in the pathophysiology of obstruction between children and adults has led to different preferred treatment modalities. While children are more likely to have an obstructive membrane blocking the natural nasal ostium at the valve of Hasner, which often resolves spontaneously or with conservative therapy (such as topical antibiotics and massage). The treatment of epiphora in adults, through medical management such as probing, irrigation, and antibiotic therapy, is often futile.

Dacryocystorhinostomy with or without silicone intubation remains the treatment of choice, but often requires general anesthesia and leaves facial scars; failure still occurs in 6% to 21 %. To overcome these drawbacks, fluoroscopic nonoperative balloon dilation and stent placement have been used in the treatment of epiphora.

Lacrimal duct stenosis is the narrowing of the tear duct that prevents the normal drainage of tears from the surface of the eye. With this phenomenon, it can lead to many different issues in the human eye, ranging from irritation in the eye and redness to a more serious infection. In some cases, if the tears are not properly draining, the stagnant tears can result in bacterial growth, fungi, and viruses. With the accumulation of bacterial growth and inflammation, this is where infection can occur.

To prevent these simple and serious issues, current procedures include placing a lacrimal stent into the lacrimal duct. This stent is intended to open up the lacrimal duct by placing outward circumferential pressure on the lacrimal duct. The placement of the stent is important to properly correct the lacrimal duct so tears can drain through the duct after stent removal months later. The way the stent is placed is through a procedure of feeding the stent into the puncta in the corner of the eye where it is lead into the lacrimal sac.

The issue with currently used stents is that the tears can only be drained by channeling the tears on the outside of the stent between the stent and the lacrimal system, instead of through the internal lumen of the stent. This is not the most effective means of transporting the fluid, as the lacrimal duct with stenosis is already narrow and the stent occupies space, thereby resulting in a restrictive passage. For some patients, the stent needs to remain in place for many months and even years until their lacrimal system is expanded.

Song pioneered the development of a stent that can be placed across an obstruction in the lacrimal system, theoretically reducing the rate of re-obstruction compared with balloon dacryocystoplasty. Therefore, making the polyurethane Song Nasolacrimal Stent the most commonly used lacrimal stent.

Song Nasolacrimal Stents introduced across the site of obstruction showed good success (93%). Suggested modifications to Song's technique included the use of a Ritleng probe to introduce the guidewire through the site of obstruction and use of a Crawford hook to retrieve the guidewire from the nose.

However, long-term success rates with the Song stent have recently been reported ranging from 50% to 86%. There appear to be three major causes for stent failure: inability to insert the stent primarily, malposition within the nasolacrimal system and obstruction by granulation tissue.

Nasolacrimal duct tubes are known from WO 2015/002510 and US 2014/364790. A tear-duct drain is known from US 2008/082037. WO 2011/075481 teaches as so-called intra-cameral sustained release device. From WO 2014/150292 drug delivery devices for the treatment of ocular disorders are known. AT 316 011 discloses a cannula tube for irrigation and threading of diseased tear ducts.

The need for new and improved stents that can mitigate the limitations of Song's technique led to the development of the present invention.

According to the principles of the present teachings, the invention relates to a lacrimal stent according to independent claim 1.

Further areas of applicability will become apparent from the description provided herein. The description and specific examples in this summary are intended for purposes of illustration only and are not intended to limit the scope of the present disclosure.

### DRAWINGS

The drawings described herein are for illustrative purposes only of selected embodiments and not all possible implementations, and are not intended to limit the scope of the present disclosure.
FIG. 1 is a side view of a lacrimal stent according to the principles of the present teachings having three exemplary axial ports exposing the internal lumen, wherein each of the ports is placed along the stent with approximately 120 degrees of rotation between two ports
FIG. 2 is a perspective view of the lacrimal stent according to the principles of the present teachings having an initial tear flow into the internal lumen of the stent.
FIG. 3 illustrates the lacrimal stent according to the principles of the present teachings having three exemplary axial ports, each placed along the stent with approximately 120 degrees of rotation between adjacent two, and further having an alignment marker helpful in the placement of the stent.
FIG. 4 illustrates the lacrimal stent according to the principles of the present teachings being used in a lacrimal drainage system of a patient.
FIG. 5 is a perspective view of the lacrimal stent according to the principles of the present teachings having rounded edges to improve patient comfort and improve tear flow into the device.

Corresponding reference numerals indicate corresponding parts throughout the several views of the drawings.

### DETAILED DESCRIPTION

Example embodiments will now be described more fully with reference to the accompanying drawings.

Example embodiments are provided so that this disclosure will be thorough, and will fully convey the scope to those who are skilled in the art. Numerous specific details are set forth such as examples of specific components, devices, and methods, to provide a thorough understanding of embodiments of the present disclosure. It will be apparent to those skilled in the art that specific details need not be employed, that example embodiments may be embodied in many different forms and that neither should be construed to limit the scope of the disclosure. In some example embodiments, well-known processes, well-known device structures, and well-known technologies are not described in detail.

The terminology used herein is for the purpose of describing particular example embodiments only and is not intended to be limiting. As used herein, the singular forms "a," "an," and "the" may be intended to include the plural forms as well, unless the context clearly indicates otherwise. The terms "comprises," "comprising," "including," and "having," are inclusive and therefore specify the presence of stated features, integers, steps, operations, elements, and/or components, but do not preclude the presence or addition of one or more other features, integers, steps, operations, elements, components, and/or groups thereof. The method steps, processes, and operations described herein are not to be construed as necessarily requiring their performance in the particular order discussed or illustrated, unless specifically identified as an order of performance. It is also to be understood that additional or alternative steps may be employed.

When an element or layer is referred to as being "on," "engaged to," "connected to," or "coupled to" another element or layer, it may be directly on, engaged, connected or coupled to the other element or layer, or intervening elements or layers may be present. In contrast, when an element is referred to as being "directly on," "directly engaged to," "directly connected to," or "directly coupled to" another element or layer, there may be no intervening elements or layers present. Other words used to describe the relationship between elements should be interpreted in a like fashion (e.g., "between" versus "directly between," "adjacent" versus "directly adjacent," etc.). As used herein, the term "and/or" includes any and all combinations of one or more of the associated listed items.

Although the terms first, second, third, etc. may be used herein to describe various elements, components, regions, layers and/or sections, these elements, components, regions, layers and/or sections should not be limited by these terms. These terms may be only used to distinguish one element, component, region, layer or section from another region, layer or section. Terms such as "first," "second," and other numerical terms when used herein do not imply a sequence or order unless clearly indicated by the context. Thus, a first element, component, region, layer or section discussed below could be termed a second element, component, region, layer or section without departing from the teachings of the example embodiments.

Spatially relative terms, such as "inner," "outer," "beneath," "below," "lower," "above," "upper," and the like, may be used herein for ease of description to describe one element or feature's relationship to another element(s) or feature(s) as illustrated in the figures. Spatially relative terms may be intended to encompass different orientations of the device in use or operation in addition to the orientation depicted in the figures. For example, if the device in the figures is turned over, elements described as "below" or "beneath" other elements or features would then be oriented "above" the other elements or features. Thus, the example term "below" can encompass both an orientation of above and below. The device may be otherwise oriented (rotated 90 degrees or at other orientations) and the spatially relative descriptors used herein interpreted accordingly.

A stable tear film is essential for good visual function of mammals, such as humans. However, epiphora is a condition describing excessive tearing. Epiphora is a very common condition that can result in loss of quality of life; interfere with activities such as driving, reading, watching TV, being outdoors, and social interactions; and become a major risk for tear duct infection (dacryocystitis), especially in children. Additionally, the constant dabbing of the cheeks can also lead to skin irritation and puts the eyes at risk of infection.

While the prevalence of epiphora is not known, the incidence of symptomatic nasolacrimal duct obstruction in 1996-2000 was 30.47 per 100,000, or approximately 120,000 new cases per year. Globally, the incidence is even higher, with case series publications from India, Singapore and South Korea suggesting a significant global disease burden.

In the treatment of epiphora, and in order to facilitate proper healing, surgeons frequently place a temporary silicone tube within the healing lacrimal drainage system to stent the passages during the healing process. Many thousands of these procedures are performed each year, and the longer the stents stay in place, the higher the likelihood of long-term surgical success. However, conventional stents occupy space within the very narrow lacrimal drainage apparatus, thereby delaying maximal improvement in tear drainage until the stent is removed weeks to months after the procedure. This issue is further exacerbated in cancer patients on chemotherapy, such as docetaxel (Taxotere) chemotherapy for breast cancer, which requires prolonged stenting.

Despite the fact that stents may have a hollow lumen, there are no known stents on the market in which the lumen is open to and can facilitate internal tear drainage - possibly because of the engineering challenge of creating a suitable opening into the lumen that is large enough to allow tears to flow in but not so large that the stent becomes too weak and fragile that it breaks prematurely. This represents a key engineering challenge in the industry that was previously unmet. With a market of approximately 120,000 procedures per year in the United States, and millions globally, and with silicone stent prices ranging from $100-500, there is an unmet need and the market to support developing a new and superior product. According to the principles of the present teachings, a stent is provided having axial ports in a stent structure that does not significantly weakening the overall structure of the lacrimal stent.

Through an active collaboration between an ophthalmologist and a mechanical engineer, the lacrimal stent of the present teachings provides an opening(s) strategically constructed to drain tears into the lumen, while optimizing the basic characteristics of the silicone tubing for the purpose of intraluminal drainage. Such a stent can revolutionize the use of stents in the treatment of symptomatic, debilitating epiphora.

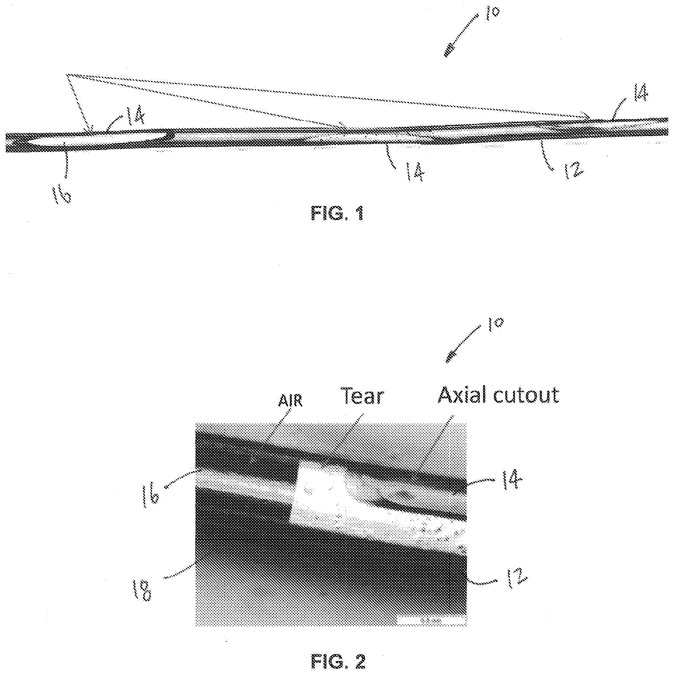

As illustrated in FIGS. 1-5, the present invention provides a lacrimal stent 10 having a generally tubular, hollow silicone structure 12 with one or more axial ports 14 designed to channel tears from the ocular surface into the hollow cavity or lumen 16 of stent 10 and down into the nasal cavity of the patient or mammal.

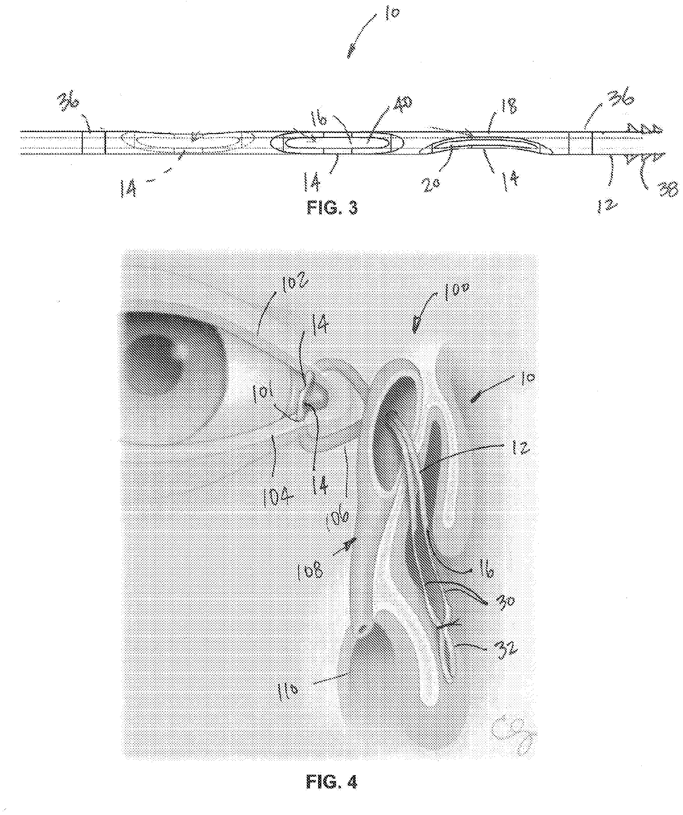

With particular reference to FIG. 4, the lacrimal drainage system 100 of a human generally extends from the small opening (puncta) 101 located on the upper 102 and lower 104 eyelid into a small tube called a canaliculus 106 which, in turn, empties into the tear sac 108 located at the inside corner of the eye. An opening in the lacrimal sac leads into the nasolacrimal duct 110, which is a canal that passes through the bony structures surrounding the nose and empties tears into the nasal cavity. As illustrated in FIG. 4, lacrimal stent 10 can be particularly placed within the lacrimal drainage system 100 such that one or more axial or ports 14 are positioned generally adjacent the corner of the eye to facilitate drainage of tears. FIG. 2 illustrates lacrimal stent 10 having a tear entering the lumen 16 from the axial port 14. In some embodiments, lacrimal stent 10 has an outer diameter on the scale of 500 to 1000 micrometers for sufficient placement within the lacrimal drainage system 100.

With continued reference to FIGS. 1-5, the lacrimal stent 10 employs axial ports 14 that are designed and created on the exterior surface 18 of structure 12 in order to effectively channel the tears into lumen 16 of lacrimal stent 10. In some embodiments, it is desirable that an opening 20 of axial ports 14 is large enough such that tears penetrate the surface of opening 20 (i.e. overcome surface tension and other barriers) with little to no resistance. However, this desire is balanced with the opposing desire to maintain structure integrity of lacrimal stent 10. To this end, opening 20 of axial ports 14 are sized, configured, and oriented to sufficiently permit fluid flow and sufficiently sized, configured, and oriented to maintain structural integrity of lacrimal stent 10.

As discussed, surface tension plays a key factor in the success of the present invention. The surface tension of the tears, when they reach the openings 20 of the axial ports 14, may hinder the ability of the tears to pass into the lumen 16 of lacrimal stent 10. In some embodiments, if openings 20 are not created to proper size for the tear to enter, then the cohesive forces will not break apart and will keep the liquid from entering inside lumen 16. It has been found that, in some embodiments, slot opening 20 with a length greater than 2 mm and/or a depth greater than about 300 micrometers will have better drainage efficiency.

To further provide sufficient fluid flow and structural integrity, according to the invention, axial ports 14 are shaped having a generally smooth, rounded, elongated U-shape such that the stress placed on lacrimal stent 10, when under tension (for example during placement or removal), is more evenly distributed through lacrimal stent 10 material. This permits lacrimal stent 10 to maintain sufficient strength so as to resist breaking during normal use. According to the invention the depth of cut of each individual axial ports 14 is between 40-65% of the diameter of lacrimal stent 10. This has been found to enable the tears to flow into lumen 16 of lacrimal stent 10 (the bigger the opening the better) while still maintaining sufficient strength.

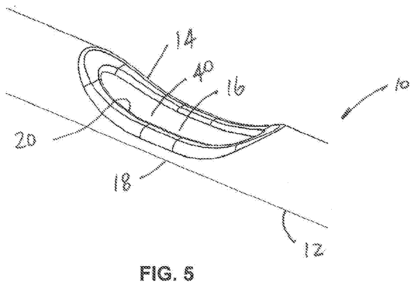

In some embodiments, as illustrated in FIG. 5, smooth edges on opening 20 of axial ports 14 further aid in minimizing stress concentrations (important for maintaining strength) and also provides a clear path for tears to flow into lumen 16 of lacrimal stent 10. Moreover, it has been found that openings 20 and/or axial ports 14 having rounded edges provide improved comfort for the patient and improved drainage of the tears through lumen 16.

In some embodiments, axial ports 14 are located at approximately 120 degree radial intervals about structure 12 so that no matter what rotational orientation lacrimal stent 10 is placed; tears will always have a clear opening to flow into. For example, if all the openings were on one side, the openings could potentially get blocked if rotated toward the corner of the eye. However, it should be understood that axial ports 14 can be located at other radial intervals, such as, but not limited to, within a range of 90 to 150 degrees.

In some embodiments, axial ports 14 are longitudinal spacing along structure 12 such that one axial ports 14 does not overlap with another axial ports 14 to help maintain the overall strength of lacrimal stent 10. If axial ports 14 were overlapped longitudinally, there could be overly narrow sections of wall of lacrimal stent 10 and failure might occur during use.

In a preferred embodiment, three axial ports 14 can span a total of 10-15mm longitudinally on lacrimal stent 10. This allows axial ports 14 to not overlap with one another yet be close enough together such that they can reside in the corner of the eye and facilitate tear drainage at any rotational orientation.

In some embodiments, lacrimal stent 10 can further include drainage cutouts 30 formed along a distal end 32 of lacrimal stent 10 to permit fluid within lumen 16 to flow therefrom.

The present invention emphasizes the correct placement of lacrimal stent 10 through a procedure of feeding lacrimal stent 10 into the puncta 101 in the corner of the eye where it is lead into the lacrimal sac, thereby facilitating efficient tear collection and drainage. In some embodiments, alignment markers 36 can be disposed on structure 12 and/or exterior surface 18 of lacrimal stent 10 to provide indicia facilitating proper placement of lacrimal stent 10 within the lacrimal drainage system 100. To this end, alignment marks 36 can be evenly placed adjacent the patient's eye lids or other physical structure to ensure optimize functionality and comfort.

During manufacturing, axial ports 14 can be produced through a number of different methods, including, but not limited to, direct injection or compression molding, razor cutting, or laser cutting. If using a razor cutting technique, a fixture is preferably created to hold lacrimal stent 10 in place. That is, the fixture can include a rectangular precision-milled fixture, preferably made from a rigid material such as stainless-steel, which includes an elongated narrow channel of varying depths that lacrimal stent 10 can be pressed into. In some embodiments, the internal width of the channel can be about 10% smaller than the outer width of lacrimal stent 10 to be cut such that lacrimal stent 10 can be pressed into the channel and held in place via an interference fit. The depth of the channel can be selected such that the sections with no desired axial ports are in a channel with a depth greater than the diameter of lacrimal stent 10 and the sections with desired axial ports are in a channel with a depth less than the diameter of lacrimal stent 10. This varying channel depth elevates the section(s) of the proposed axial port segment(s) ("protruding stent segment") partially above the flat top surface of the fixture while allowing the desired unmodified section(s) to reside below the flat top surface of the fixture. A cutting apparatus, such as a razor blade, can then be slid across the top surface of the fixture, allowing for smooth and precise cutting of the selectively raised sections.

In some embodiments, the rounded edges of opening 20 can be created concurrently with axial ports 14. However, it should be understood that these features could be molded in, cut with a specific laser pattern, or created using cutting fixture/blade specific to the rounded shape of the axial port 14.

The present invention overcomes the drawback of currently used stents (that channel the tears on the outside of the silicone tube) by facilitating efficient channeling of tears through the lumen 16 of lacrimal stent 10. The conventional technique was not an effective means of transporting the fluid as the lacrimal duct with stenosis is already narrow and lacrimal stent 10 occupies space. For some patients, lacrimal stent 10 needs to remain in place for many months and even years, and allowing for tear flow through the lumen of lacrimal stent 10 will improve epiphora symptoms.

| | |
|---|---|
| **Description of Device** | • A hollow tubular lacrimal stent (i.e. bicanalicular nasolacrimal duct stents) with at least one axial port exposing the internal lumen |
| | • Each axial port allows drainage tears from the ocular surface, into the internal lumen of the stent, and into the nasal/oropharynx cavities |
| | • Each axial port is included to further improve drainage of tears into the lumen of the stent. These multiple ports can also be placed at different angles further reducing the likelihood that all three openings are blocked by the eye. |
| | • Alignment markers can also be added to the stent, helping to facilitate proper placement of the device such that the axial ports are positioned at the inside corner of the eye |
| **Clinical Indications For Use** | • Patients experiencing epiphora secondary to insufficient tear drainage, caused by punctal/canalicular/nasolacrimal duct stenosis |
| **Process For Using Device** | **•** Operating room placement of bicanalicular nasolacrimal duct stent under anesthesia. After anesthesia induction and draping of the face, the puncta are dilated with an instrument, and then the canaliculi and nasolacrimal duct are probed and dilated with Bowman probes. The stents are placed sequentially through the punta, canaliculi and duct and retrieved in the nose using a hook or hemostat. The stent is aligned such that the axial port(s) are exposed at the inside corner of the eye. The stent is then cut to size. |
| **Anticipated Outcomes** | • Reduced epiphora with improved symptoms of tearing down the cheek, fogging of vision. |
| **Anticipated Safety** | • Excellent safety profile. - as safe as current standard-of-care bicanalicular nasolacrimal duct stents. Safety would involve risk of punctal injury, conjunctival/corneal abrasion, and infection. |

It should be understood that variations of the present teachings are anticipated. For example, to prevent lacrimal stent 10 from moving out of place, ridges 38 (FIG. 3) could be molded into the exterior surface 18 of lacrimal stent 10, similarly to a plurality of stair-like fish hooks. This could help prevent lacrimal stent 10 from being pulled one way or the other, but would still allow for removal since the ridges 38 would create a one-way removal interface. Moreover, in some embodiments, a surface coating or texture 40 (such as a hydrophilic and/or hydrophobic coating) could be added at least on the inside of lacrimal stent 10 to facilitate increased tear flow rate. Still further, in some embodiments, axial ports 14 can be configured as a porous material having a plurality of openings formed in an open cell matrix to facilitate the flow of tears inside the lumen 16 of lacrimal stent 10 and maintain structural integrity.

## Claims

1. A lacrimal stent (10) for intraluminal drainage of a mammal, the mammal having a lacrimal drainage structure, the lacrimal stent (10) comprising:
an elongated tubular silicone member (12) configured to be inserted into at least a portion of the lacrimal drainage structures of the mammal, the elongated tubular silicone member (12) having an exterior sidewall surface (18) and an interior lumen (16); and
at least one axial port (14) extending through the exterior sidewall surface (18) to the lumen (16) of the elongated tubular silicone member (12), each axial port (14) is configured to permit fluidic flow of the tears of the mammal into the lumen (16) for the purpose of intraluminal drainage, wherein the depth of cut of each axial port (14) is between 40-65% of the diameter of lacrimal stent (10), and wherein each axial port (14) is shaped having a generally smooth, rounded, elongated U-shape.

2. The lacrimal stent (10) according to Claim 1, wherein the at least one axial port (14) comprises a plurality of axial ports (14), each of the plurality of axial ports (14) being spaced at radial offset positions about the elongated tubular silicone member (12).

3. The lacrimal stent (10) according to Claim 2, wherein each of the plurality of axial ports (14) is spaced at radial offset positions 120 degrees relative to adjacent axial ports (14).

4. The lacrimal stent (10) according to Claim 2, wherein each of the plurality of axial ports (14) is spaced at radial offset positions 90 to 150 degrees relative to adjacent axial ports (14).

5. The lacrimal stent (10) according to Claim 1, wherein the at least one axial port (14) comprises a plurality of axial ports (14), each of the plurality of axial ports (14) being spaced at longitudinal offset positions along the elongated tubular silicone member (12).

6. The lacrimal stent (10) according to Claim 1 further comprising:
at least one alignment indicia formed on the exterior surface (18) of the elongated tubular silicone member (12) to facilitate alignment of the lacrimal stent (10) within the lacrimal drainage structure.

7. The lacrimal stent (10) according to Claim 6 wherein the at least one alignment indicia comprises an alignment marker formed on the exterior surface (18) of the elongated tubular silicone member (12).

8. The lacrimal stent (10) according to Claim 1 wherein the elongated tubular silicone member (12) is configured to be inserted into at least one of a punctum, canaliculus, and nasolacrimal duct of the lacrimal drainage structure of the mammal.

9. The lacrimal stent (10) according to Claim 1 wherein the internal lumen (16) of the elongated tubular silicone member (12) comprises a coating or texture surface (40).

10. The lacrimal stent (10) according to Claim 1 wherein the internal lumen (16) of the elongated tubular silicone member (12) comprises a hydrophilic coating.

11. The lacrimal stent (10) according to Claim 1 further comprising:
one or more ridges (38) formed on the exterior sidewall surface (18) of the elongated tubular silicone member (12) configured to engage within the lacrimal drainage structure.

## Patentansprüche

1. Lakrimaler Stent (10) zur intraluminalen Drainage eines Säugetiers, wobei das Säugetier eine lakrimale Drainagestruktur aufweist, wobei der lakrimale Stent (10) Folgendes aufweist:
ein längliches, röhrenförmiges Silikonelement (12), das derart ausgebildet ist, dass es in zumindest einen Abschnitt der lakrimalen Drainagestrukturen des Säugetiers eingesetzt werden kann, wobei das längliche, röhrenförmige Silikonelement (12) eine äußere Seitenwandoberfläche (18) und ein inneres Lumen (16) aufweist; und
zumindest eine axiale Öffnung (14), die sich durch die äußere Seitenwandoberfläche (18) zum Lumen (16) des länglichen, röhrenförmigen Silikonelements (12) erstreckt, wobei jede axiale Öffnung (14) derart ausgebildet ist, dass sie einen Fluidstrom der Tränen des Säugetiers in das Lumen (16) zum Zweck der intraluminalen Drainage ermöglicht, wobei die Schnitttiefe jeder axialen Öffnung (14) zwischen 40-65% des Durchmessers des lakrimalen Stents (10) beträgt, und wobei jede axiale Öffnung (14) eine generell glatte, abgerundete, längliche U-Form aufweist.

2. Lakrimaler Stent (10) gemäß Anspruch 1, wobei die zumindest eine axiale Öffnung (14) eine Vielzahl von axialen Öffnungen (14) aufweist, wobei jede der Vielzahl von axialen Öffnungen (14) an radial versetzten Positionen um das längliche, röhrenförmige Silikonelement (12) beabstandet ist.

3. Lakrimaler Stent (10) gemäß Anspruch 2, wobei jede der Vielzahl von axialen Öffnungen (14) in radial versetzten Positionen um 120 Grad relativ zu benachbarten axialen Öffnungen (14) beabstandet ist.

4. Lakrimaler Stent (10) gemäß Anspruch 2, wobei jede der Vielzahl von axialen Öffnungen (14) in radial versetzten Positionen von 90 bis 150 Grad relativ zu benachbarten axialen Öffnungen (14) beabstandet ist.

5. Lakrimaler Stent (10) gemäß Anspruch 1, wobei die zumindest eine axiale Öffnung (14) eine Vielzahl von axialen Öffnungen (14) aufweist, wobei jede der Vielzahl von axialen Öffnungen (14) an in Längsrichtung versetzten Positionen entlang des länglichen, rohrförmigen Silikonelements (12) beabstandet ist.

6. Lakrimaler Stent (10) gemäß Anspruch 1, der ferner Folgendes aufweist:
zumindest eine auf der äußeren Oberfläche (18) des länglichen, röhrenförmigen Silikonelements (12) gebildete Ausrichtungsindizes, um die Ausrichtung des lakrimalen Stents (10) innerhalb der lakrimalen Drainagestruktur zu erleichtern.

7. Lakrimaler Stent (10) gemäß Anspruch 6, wobei die zumindest einen Ausrichtungsindizes eine Ausrichtungsmarkierung aufweisen, die auf der äußeren Oberfläche (18) des länglichen, röhrenförmigen Silikonelements (12) gebildet ist.

8. Lakrimaler Stent (10) gemäß Anspruch 1, wobei das längliche, röhrenförmige Silikonelement (12) derart ausgebildet ist, dass es in zumindest eines von Punctum, Canaliculus und Ductus nasolacrimalis der lakrimalen Drainagestruktur des Säugetiers eingeführt werden kann.

9. Lakrimaler Stent (10) gemäß Anspruch 1, wobei das innere Lumen (16) des länglichen, röhrenförmigen Silikonelements (12) eine Beschichtung oder eine texturierte Oberfläche (40) aufweist.

10. Lakrimaler Stent (10) gemäß Anspruch 1, wobei das innere Lumen (16) des länglichen, röhrenförmigen Silikonelements (12) eine hydrophile Beschichtung aufweist.

11. Lakrimaler Stent (10) gemäß Anspruch 1, der ferner Folgendes aufweist:
eine oder mehrere Rippen (38), die an der äußeren Seitenwandoberfläche (18) des länglichen, röhrenförmigen Silikonelements (12) gebildet sind und derart ausgebildet sind, dass sie in der lakrimalen Drainagestruktur zusammenwirken.

## Revendications

1. Endoprothèse lacrymale (10) pour le drainage intraluminal d'un mammifère, le mammifère ayant une structure de drainage lacrymal, l'endoprothèse lacrymale (10) comprenant :
un élément en silicone tubulaire allongé (12) configuré pour être inséré dans au moins une partie des structures de drainage lacrymal du mammifère, l'élément en silicone tubulaire allongé (12) ayant une surface de paroi latérale extérieure (18) et une lumière intérieure (16) ; et
au moins un orifice axial (14) s'étendant à travers la surface de paroi latérale extérieure (18) jusqu'à la lumière (16) de l'élément en silicone tubulaire allongé (12), chaque orifice axial (14) est configuré pour permettre l'écoulement fluidique des larmes du mammifère dans la lumière (16) à des fins de drainage intraluminal, dans laquelle la profondeur de coupe de chaque orifice axial (14) est comprise entre 40 et 65% du diamètre de l'endoprothèse lacrymale (10), et dans laquelle chaque orifice axial (14) est conformé de manière à avoir une forme en U globalement lisse, arrondie et allongée.

2. Endoprothèse lacrymale (10) selon la revendication 1, dans laquelle l'au moins un orifice axial (14) comprend une pluralité d'orifices axiaux (14), chacun de la pluralité d'orifices axiaux (14) étant espacé à des positions radiales décalées autour de l'élément en silicone tubulaire allongé (12).

3. Endoprothèse lacrymale (10) selon la revendication 2, dans laquelle chacun de la pluralité d'orifices axiaux (14) est espacé à des positions radiales décalées de 120 degrés par rapport à des orifices axiaux adjacents (14).

4. Endoprothèse lacrymale (10) selon la revendication 2, dans laquelle chacun de la pluralité d'orifices axiaux (14) est espacé à des positions radiales décalées de 90 à 150 degrés par rapport à des orifices axiaux adjacents (14).

5. Endoprothèse lacrymale (10) selon la revendication 1, dans laquelle l'au moins un orifice axial (14) comprend une pluralité d'orifices axiaux (14), chacun de la pluralité d'orifices axiaux (14) étant espacé à des positions longitudinales décalées le long de l'élément en silicone tubulaire allongé (12).

6. Endoprothèse lacrymale (10) selon la revendication 1, comprenant en outre :
au moins un repère d'alignement formé sur la surface extérieure (18) de l'élément en silicone tubulaire allongé (12) pour faciliter l'alignement de l'endoprothèse lacrymale (10) à l'intérieur de la structure de drainage lacrymal.

7. Endoprothèse lacrymale (10) selon la revendication 6, dans laquelle l'au moins un repère d'alignement comprend une marque d'alignement formée sur la surface extérieure (18) de l'élément en silicone tubulaire allongé (12).

8. Endoprothèse lacrymale (10) selon la revendication 1, dans laquelle l'élément en silicone tubulaire allongé (12) est configuré pour être inséré dans au moins l'un parmi un point lacrymal, un canal d'union et un conduit lacrymonasal de la structure de drainage lacrymal du mammifère.

9. Endoprothèse lacrymale (10) selon la revendication 1, dans laquelle la lumière interne (16) de l'élément en silicone tubulaire allongé (12) comprend une surface de revêtement ou texturée (40).

10. Endoprothèse lacrymale (10) selon la revendication 1, dans laquelle la lumière interne (16) de l'élément en silicone tubulaire allongé (12) comprend un revêtement hydrophile.

11. Endoprothèse lacrymale (10) selon la revendication 1 comprenant en outre :
une ou plusieurs crêtes (38) formées sur la surface de paroi latérale extérieure (18) de l'élément en silicone tubulaire allongé (12) configurées pour s'engager à l'intérieur de la structure de drainage lacrymal.
